# EUROPEAN PATENT APPLICATION

(11) **EP 4 582 409 A1**
(43) Date of publication of application: **09.07.2025**
(21) Application number: 23867491.5
(22) Date of filing: 19.09.2023
(51) Int. Cl.: C07C 229/12, A61K 47/18, A61P 37/04

(54) **METHOD FOR PREPARING 2-HYDROXYETHYL AMINOCAPROATE COMPOUND AND USE THEREOF**

(30) Priority: 19.09.2022 CN 202211137835
(71) Applicant: Suzhou Suncadia Biopharmaceuticals Co., Ltd., Suzhou, Jiangsu 215126 (CN); Jiangsu Hengrui Pharmaceuticals Co., Ltd., Lianyungang, Jiangsu 222047 (CN)
(72) Inventor: GU, Yueqing, Suzhou, Jiangsu 215126 (CN); FANG, Xinxin, Suzhou, Jiangsu 215126 (CN); LU, Gang, Suzhou, Jiangsu 215126 (CN); LI, Zhiya, Suzhou, Jiangsu 215126 (CN)
(74) Representative: Potter Clarkson
(86) International application number: PCT/CN2023/119665
(87) International publication number: WO 2024/061204

(57) **Abstract**

The present disclosure relates to a method for preparing a 2-hydroxyethyl aminocaproate compound and use thereof. Specifically, the present disclosure provides a method for preparing a compound of formula (I) or a salt thereof. The method comprises the step of reacting a compound of formula (A) with a compound of formula (B) to form a compound of formula I. The overall process is simple and convenient to operate, the yield is high, the quality of an obtained sample is excellent, and thus the method is suitable for large-scale production.

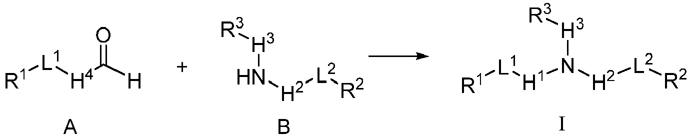

## Description

### FIELD OF THE INVENTION

The present invention relates to a method for preparing 2-hydroxyethyl aminocaproate compound and use thereof.

### BACKGROUND OF THE INVENTION

Currently, the delivery systems for nucleic acid drugs can be divided into two categories of viral vector systems and non-viral systems. Liposome-mediated delivery for nucleic acid drugs is the main means of the non-viral delivery systems.

Various challenges need to be addressed to deliver an *in vitro* synthesized mRNA into cells for translation and expression of target proteins, and the first priority is to solve the enzymatic degradation and the cell membrane barrier involved in delivery of mRNA to cells. Both of the two mRNA vaccines currently approved for COVID-19 utilize a LNP delivery system to achieve delivery of mRNA. The LNP delivery system may not only efficiently deliver mRNA to specific target cells and prevent mRNA from degradation, but also help mRNA escape from the endosome into the cytoplasm. The ionizable lipid employed by the company Moderna is SM-102, and the ionizable lipid employed by the company Pfizer is ALC-0315.

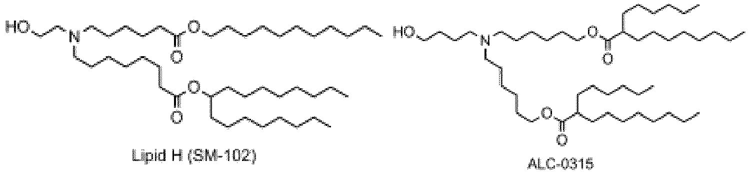

CN202210651178.7 has reported a novel class of ionizable lipids, represented by compound 1, which exhibits a superior delivery effect. Meanwhile, the prepared liposomes will be distributed more in the liver and less in the spleen, thereby having better organ targeting ability, 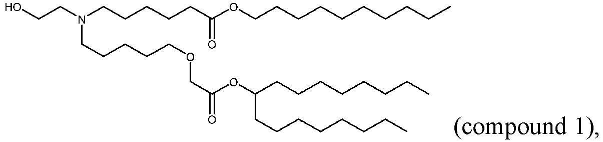 the preparation method of which is generally as follows:

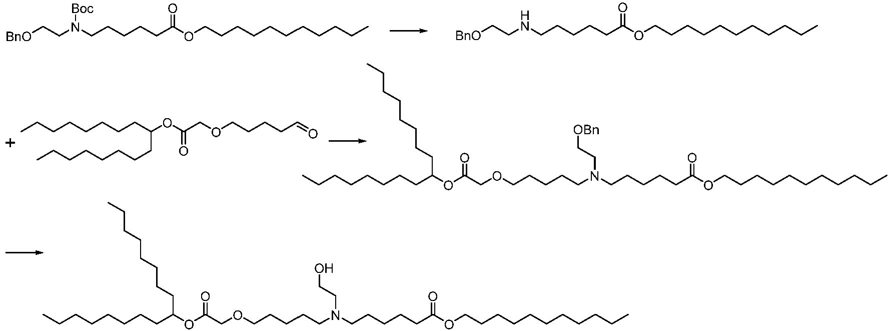

There are additional common methods for preparing an ionizable lipid: 1) US10106490 has reported a method for preparing an ionizable lipid by SN1 nucleophilic reaction between an amino alcohol and bromo-ester,

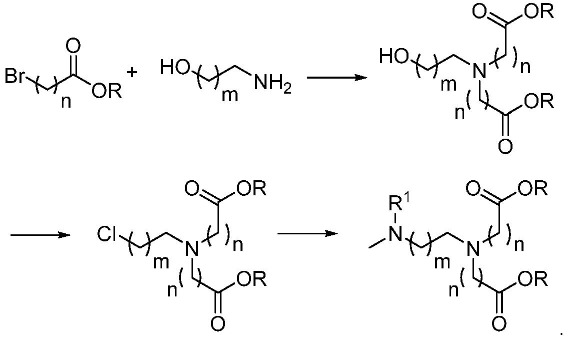

2) WO2018170306 also has reported a similar reaction process, i.e., a method in which the SN1 reaction of Compound f2 with an amino alcohol is performed to obtain the ionizable lipid h2,

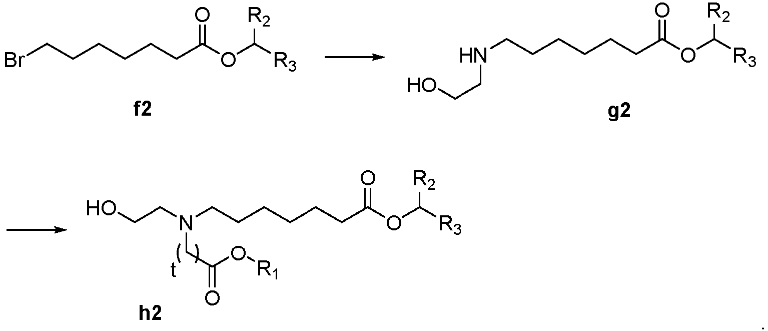

### SUMMARY OF THE INVENTION

The disclosure provides a method for preparing a compound of formula I or a pharmaceutically acceptable salt thereof, 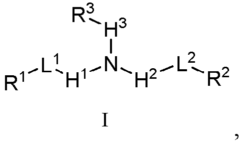 comprising a step of reacting a compound of formula A with a compound of formula B to form a compound of formula I, 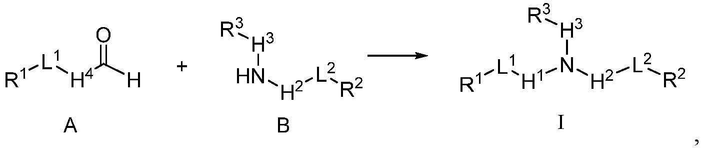 wherein L¹ and L² are each independently selected from the group consisting of -C(O)O-, -OC(O)-, -C(O)- and -OC(O)O-;
H¹ and H² are each independently selected from the group consisting of C₁₋₁₂heteroalkylene, C₁₋₁₂ alkylene and C₂₋₁₂ alkenylene;
H³ is selected from the group consisting of C₁₋₂₄ alkylene, C₂₋₂₄ alkenylene, C₃₋₈ cycloalkylene and C₃₋₈ cycloalkenylene;
H⁴ is selected from the group consisting of C₂₋₁₁ heteroalkylene, C₂₋₁₁ alkyleneand C₃₋₁₁ alkenylene;
R¹ and R² are each independently selected from the group consisting of C₁₋₂₄ alkyl and C₂₋₂₄ alkenyl;
R³ is selected from the group consisting of -CN, -C(O)OR⁴, -OC(O)R⁴, -OR⁵ and -NR⁵C(O)R⁴, R⁴ is selected from the group consisting of C₁₋₆ alkyl and C₂₋₆ alkenyl, and R⁵ is selected from the group consisting of hydrogen, C₁₋₆ alkyl and C₂₋₆ alkenyl.

In some embodiments, the compound of formula A is reacted with the compound of formula B in the presence of a reducing agent selected from the group consisting of sodium borohydride and sodium triacetoxyborohydride.

Further, in some embodiments, the reaction also includes a base including, but not limited to, *N,* N-diisopropylethylamine.

In some other embodiments, the compound of formula I is a compound of formula I-1, 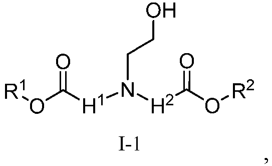 and the method comprises a step of reacting a compound of formula A-1 with a compound of formula B-1, 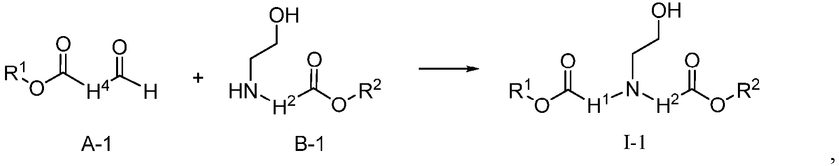 wherein H¹, H², H⁴, R¹ and R² are as defined in the compound of formula I.

In some embodiments, at least one of H¹ and H² in the compound of formula I or formula II is C₁₋₁₂ heteroalkylene, including C₂₋₉ heteroalkylene, C₃ heteroalkylene, C₄ heteroalkylene, C₅ heteroalkylene, C₆ heteroalkylene, C₇ heteroalkylene, C₈ heteroalkylene, C₉ heteroalkylene, C₁₀ heteroalkylene, C₁₁ heteroalkylene or C₁₂ heteroalkylene. In some embodiments, at least one of H¹ and H² in the compound of formula I or formula II is C₂₋₉ heteroalkylene, preferably C₂₋₅ heteroalkylene.

In some embodiments, at least one of H¹ and H² in the compound of formula I or formula II is C₂₋₉ heteroalkylene containing at least one O heteroatom.

In some embodiments, at least one of H¹ and H² in the compound of formula I or formula II is C₂₋₉ heteroalkylene containing one O heteroatom.

In some other embodiments, R¹ in the compound of formula I or formula II is selected from the group consisting of 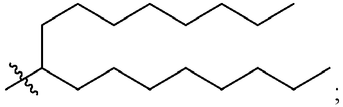 H¹ is selected from the group consisting of C₂₋₉ heteroalkylene.

In some other embodiments, R¹ in the compound of formula I or formula II is selected from the group consisting of 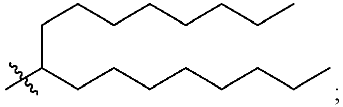 H¹ is selected from the group consisting of C₂₋₉ heteroalkylene containing one O heteroatom.

In some other embodiments, R¹ in the compound of formula I or formula II is selected from the group consisting of 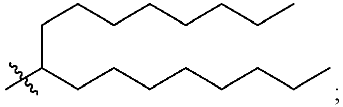 H¹ is selected from the group consisting of C₂₋₉ heteroalkylene containing one O heteroatom; R² is selected from the group consisting of C₁₋₂₄ alkyl; H² is selected from the group consisting of C₂₋₉ alkylene.

In some other embodiments, R² in the compound of formula I or formula II is selected from the group consisting of 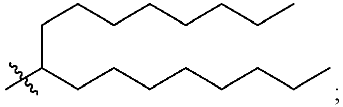 H² is selected from the group consisting of C₂₋₉ heteroalkylene.

In some other embodiments, R² in the compound of formula I or formula II is selected from the group consisting of 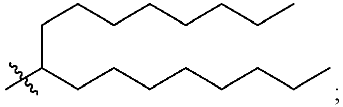 H² is selected from the group consisting of C₂₋₉ heteroalkylene containing one O heteroatom.

In some other embodiments, R² in the compound of formula I or formula II is selected from the group consisting of 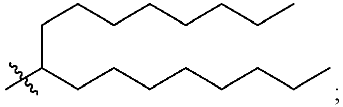 H² is selected from the group consisting of C₂₋₉ heteroalkylene containing one O heteroatom; R¹ is selected from the group consisting of C₁₋₂₄ alkyl; H¹ is selected from the group consisting of C₂₋₉ alkylene.

In another aspect, in some embodiments, R¹ in the compound of formula I or formula II is selected from the group consisting of 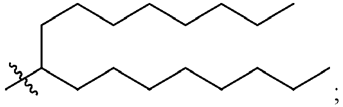 H¹ is selected from the group consisting of C₂₋₉ alkylene; R² is selected from the group consisting of C₁₋₂₄ alkyl; H² is selected from the group consisting of C₂₋₉ alkylene.

In some embodiments, R² in the compound of formula I or formula II is selected from the group consisting of 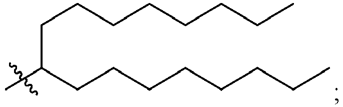 H² is selected from the group consisting of C₂₋₉ alkylene; R¹ is selected from the group consisting of C₁₋₂₄ alkyl; H¹ is selected from the group consisting of C₂₋₉ alkylene.

In some embodiments, the compound of formula I is a compound of formula I-2, 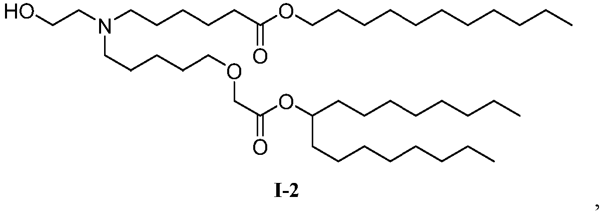 and the method comprises a step of reacting a compound of formula A-2a with a compound of formula B-2a, 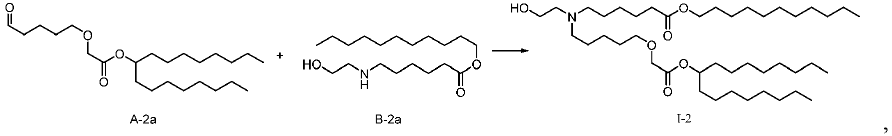 or, a step of reacting a compound of formula A-2b with a compound of formula B-2b,

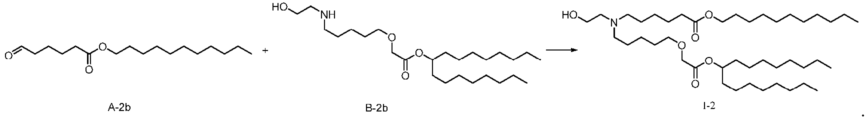

Further, the method for preparing a compound of formula I or a pharmaceutically acceptable salt thereof also comprises a step of converting a compound of formula B-2a5 to the compound of formula B-2a, 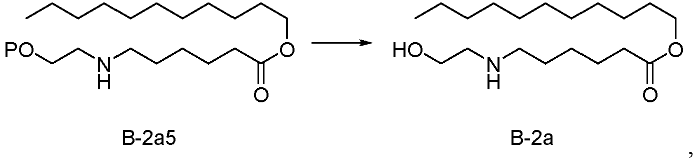 wherein, P is a protecting group for hydroxy, for example a benzyl or benzoyl group.

In some embodiments, the condition for removing the protecting group for hydroxy is a hydrogenation condition which comprising a Pd/C catalyst.

In some other embodiments, the method for preparing a compound of formula I or a pharmaceutically acceptable salt thereof also comprises a step of converting a compound of formula B-2b5 to a compound of formula B-2b, 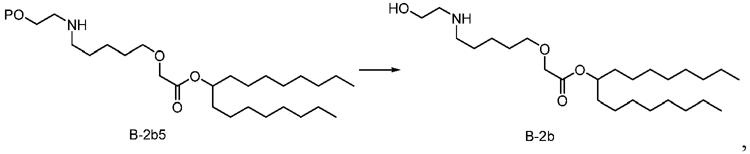 wherein, P is a protecting group for hydroxy, for example a benzyl or benzoyl group.

In some embodiments, the method for preparing a compound of formula I or a pharmaceutically acceptable salt thereof comprises forming a compound of formula B-2a from a compound of formula B-2a5 under a hydrogenation condition, and subsequently reacting the compound of formula B-2a with a compound of formula A-2a to form a compound of formula I-2,

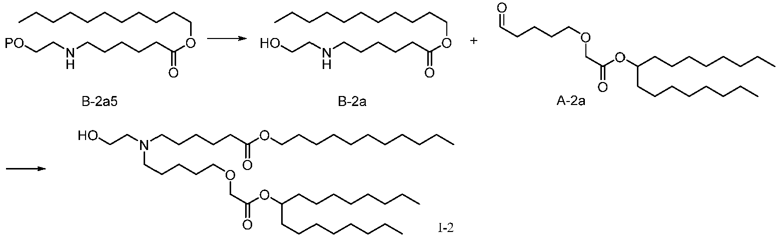

In another aspect, in some embodiments, the compound of formula I is a compound of formula I-3, 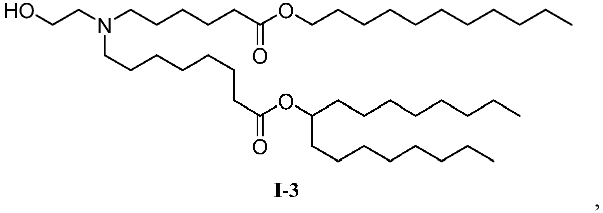 and the method comprises a step of reacting a compound of formula A-3a with a compound of formula B-3a,

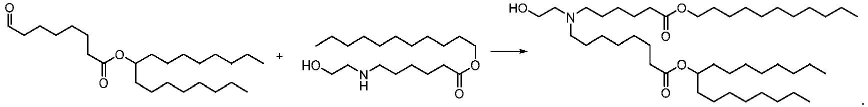

The present disclosure also provides a pharmaceutical composition comprising a compound of formula I or a pharmaceutically acceptable salt thereof prepared by the aforementioned method, and one or more pharmaceutically acceptable excipients.

**In** some embodiments, the pharmaceutical composition further comprises an active agent selected from, but not limited to, a nucleotide or nucleic acid such as mRNA.

**In** another aspect, the present disclosure also provides use of a compound of formula I or a pharmaceutically acceptable salt thereof prepared by the aforementioned method or the aforementioned pharmaceutical composition in the preparation of a medicament for the prevention and/or treatment of an immune response induced in a subject.

In another aspect, the present disclosure also provides use of a compound of formula I or a pharmaceutically acceptable salt thereof prepared by the aforementioned method or the aforementioned pharmaceutical composition in the preparation of a medicament for the prevention and/or treatment of a disease or disorder related to overexpression of polypeptide.

In another aspect, the present disclosure also provides use of a compound of formula I or a pharmaceutically acceptable salt thereof prepared by the aforementioned method or the aforementioned pharmaceutical composition in the preparation of a medicament for the prevention and/or treatment of a disease or disorder related to underexpression of polypeptide.

The method described in the present disclosure also comprises post-processing operations, for example, one or more steps of filtration, extraction, concentration, column chromatography or chiral separation, so as to obtain pure target products.

The salts of compounds described in the present disclosure include "acid" addition salts and "base" addition salts. For example, the salts are formed by acid-base reactions of acids with basic groups (amino groups), and the acids include organic or inorganic acids. In addition, the salts of compounds also include salts formed by quaternization with basic groups (amino groups), and the reagents for quaternization include linear or branched chlorohydrocarbon.

Unless otherwise stated, the terms used in the present disclosure have the following meanings.

The term "pharmaceutical composition" represents a mixture containing one or more compounds described herein or physiologically/pharmaceutically acceptable salts or prodrugs thereof and other chemical components, as well as other components such as physiologically/pharmaceutically acceptable carriers and excipients. The purpose of a pharmaceutical composition is to facilitate administration of a drug to an organism, which is conducive to absorption of an active ingredient and thus exertion of biological activity.

The term "pharmaceutically acceptable excipient" includes, but is not limited to, any adjuvant, carrier, excipient, glidant, sweetener, diluent, preservative, dye/colorant, flavor enhancer, surfactant, wetting agent, dispersant, suspending agent, stabilizer, isotonic agent, solvent or emulsifier that has been approved by the U.S. Food and Drug Administration and is acceptable for use in humans or livestock animals.

The term "effective amount" or "effectively therapeutic amount" described in the present disclosure includes an amount sufficient to improve or prevent symptoms or conditions of a medical disease. The effective amount also means an amount sufficient to permit or facilitate diagnosis. The effective amount for specific patients or veterinary subjects may vary depending on factors such as the condition to be treated, the overall health status of the patient, the route and dosage of administration, and the severity of side effects. The effective amount can be the maximum dose or dosing regimen that avoids significant side effects or toxic effects.

The term "nucleic acid" refers to a polymer composed of nucleotides, for example deoxyribonucleotides (DNA) or ribonucleotides (RNA).

The term "oligonucleotide" represents a single- or double-stranded nucleotide multimer with a length of 2 to 100 nucleotides. The term "polynucleotide" refers to a single- or double-stranded polymer composed of nucleotide monomers. In some embodiments, the length of the polynucleotide is such that it is composed of 100 or more nucleotides.

In some other embodiments, exemplary polynucleotides include, but are not limited to, mRNA.

The term "alkyl" refers to a saturated aliphatic hydrocarbon group, including linear and branched alkyl groups. In some embodiments, the alkyl group has 1-4 carbon atoms which is also known as C₁₋₄ alkyl. In some embodiments, the alkyl group has 10-22 carbon atoms which is also known as C₁₀₋₂₂ alkyl. In some embodiments, the alkyl group has 4-22 carbon atoms which is also known as C₄₋₂₂ alkyl.

The alkyl group can be unsubstituted or substituted with one or more groups selected from the group consisting of halogen, hydroxy, amino, oxo, alkyl, alkoxycarbonyl, acylamino, alkyl acylamino, dialkyl acylamino, nitro, amino, alkylamino, dialkylamino, carboxyl, thio, and thioalkyl.

The term "heteroalkyl" refers to a linear or branched alkyl group which preferably has 1 to 14 carbon atoms, more preferably 2 to 10 carbon atoms, in the chain, in which one or more carbon atoms are replaced with heteroatoms selected from S, O, and N. Exemplary heteroalkyl groups include alkyl ethers, secondary and tertiary alkylamines, amides, alkyl sulfides, and the like.

The heteroalkyl group can be unsubstituted or substituted with one or more groups selected from the group consisting of halogen, hydroxy, alkyl, amino, oxo, alkoxycarbonyl, acylamino, alkyl acylamino, dialkyl acylamino, nitro, amino, alkylamino, dialkylamino, carboxyl, thio, and thioalkyl.

The term "alkenyl" refers to an unsaturated aliphatic hydrocarbon group, including linear and branched alkenyl groups. In some embodiments, the alkenyl group has 1-4 carbon atoms which is also known as C₁₋₄ alkenyl. In some embodiments, the alkenyl group has 10-22 carbon atoms and which also known as C₁₀₋₂₂ alkenyl. In some embodiments, the alkenyl group has 4-22 carbon atoms which is also known as C₄₋₂₂ alkenyl. Exemplary alkenyl groups include vinyl, propenyl, n-butenyl, isobutenyl, 3-methylbut-2-enyl, n-pentenyl, heptenyl, octenyl, cyclohexyl-butenyl, and decenyl.

The alkenyl group can be unsubstituted or substituted with one or more groups selected from the group consisting of halogen, hydroxy, amino, oxo, alkyl, alkoxycarbonyl, acylamino, alkyl acylamino, dialkyl acylamino, nitro, amino, alkylamino, dialkylamino, carboxyl, thio, and thioalkyl.

The term "monovalent group" means that one monovalent atom or group is "formally" eliminated from a compound. The term "ene" means that two monovalent atoms or groups of atoms are "formally" eliminated from a compound, or one bivalent atom or group of atoms is "formally" eliminated from the compound.

The term "alkylene" represents the remaining moiety of an alkane molecule after removing 2 hydrogen atoms. In some embodiments, the alkylene group has 1-4 carbon atoms which is also known as C₁₋₄ alkylene. In some embodiments, the alkyl group has 10-22 carbon atoms which is also known as C₁₀₋₂₂ alkylene. In some embodiments, the alkyl group has 4-22 carbon atoms which is also known as C₄₋₂₂ alkylene.

Similarly, the definitions of "alkyleneoxy", "heteroalkylene", "alkenylene", "alkenyleneoxy", "cycloalkylene", and "heterocycloalkylene" are as that of "alkylene".

The expressions "to form" and "converting to" in the present disclosure do not specifically mean that the conversion reaction between two substrates is a single-step reaction, but can be a single-step or multi-step reaction between two substrates. If an intermediate contains a protecting group for amino, the intermediate undergoes one step of deprotecting the protected amino and then reacts with a corresponding substrate to obtain a corresponding target product.

The term "protecting group for hydroxy" is a suitable group known in the art for protecting hydroxy, as described in the literature "Protective Groups in Organic Synthesis", 5Th Ed. T. W. Greene & P. G. M. Wuts. As an example, it includes, but is not limited to, benzyl or benzoyl.

The term "excipient" in the present disclosure includes, but is not limited to, any adjuvant, carrier, glidant, sweetener, diluent, preservative, dye/colorant, flavor enhancer, surfactant, wetting agent, dispersant, suspending agent, stabilizer, isotonic agent or emulsifier that has been approved by the U.S. Food and Drug Administration and is acceptable for use in humans or livestock animals.

The structures of the compounds are identified by nuclear magnetic resonance (NMR) and/or mass spectrometry (MS). NMR shift (δ) is given in units of 10⁻⁶ (ppm). NMR is determined on a Bruker AVANCE-400 nuclear magnetic spectrometer. The solvents for determination are deuterated dimethyl sulfoxide (DMSO-*d₆*), deuterated chloroform (CDCl₃), and deuterated methanol (CD₃OD), and the internal standard is tetramethylsilane (TMS).

HPLC is determined using an Agilent 1200 high pressure liquid chromatograph (Evo C18 4.6*250 mm, 5 um column, or Xtimate C18 2.1*30 mm column).

Yantai Huanghai HSGF254 or Qingdao GF254 silica gel plates are used for the thin-layer chromatography. For the gel plates used, the specification is 0.15 mm - 0.2 mm for thin-layer chromatography (TLC), and 0.4 mm - 0.5 mm for separation and purification of products by thin-layer chromatography.

Combiflash Rf150 (TELEDYNE ISCO) or Isolara one (Biotage) is used as the rapid column purification system.

### DETAILED DESCRIPTION OF THE INVENTION

The present disclosure will be illustrated in detail in combination with the specific examples below, so as to be more fully understood by those skilled in the art. The specific examples are merely intended to explain the technical solutions of the present disclosure, and do not limit the present disclosure in any way.

### Example 1

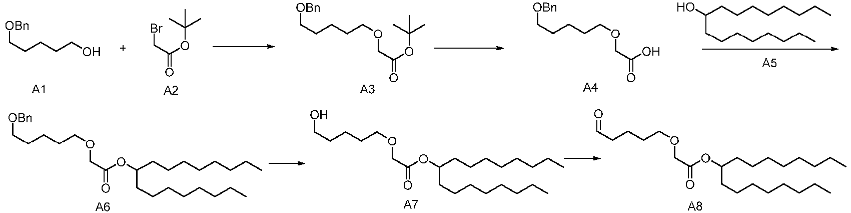

### Step 1:

Toluene and A1 (8 g) were added to NaOH aqueous solution at 0-5°C, and stirred for 1 hour at room temperature. Tert-butyl bromoacetate was slowly added dropwise thereto, and the reaction was stirred under heating overnight. Water was added, and the resulting mixture was extracted three times with methyl tert-butyl ether. The above organic phases were combined and washed three times with water. The aqueous phases were combined. The aqueous phase was directly used for the next step.

### Step 2:

Concentrated hydrochloric acid was slowly added under stirring to adjust pH to 3. The resulting mixture was extracted with DCM. The above organic phases were combined, washed with saturated saline solution, and concentrated under reduced pressure. The crude product was purified by column chromatography to obtain A4, with a two-step yield of 90%.

### Step 3:

Compound A4 was dissolved in dichloromethane and cooled to 0-5°C. Heptadecan-9-ol (A5), DMAP and EDCI were added thereto successively and stirred. The ice bath was removed, and the reaction was performed overnight at room temperature. After the complete reaction of heptadecan-9-ol monitored by TLC, water was added to the reaction solution and the solution was partitioned. The aqueous phase was extracted with DCM. The organic phases were combined, washed with saturated sodium bicarbonate aqueous solution, washed again with saturated saline solution, and concentrated under reduced pressure to obtain a crude product. The crude product was purified by column chromatography to obtain A6, with a yield of 95%.

### Step 4:

Compound A6 was dissolved in THF, and Pd/C (10%) was added thereto. Hydrogen gas was introduced to be pressurized to 0.5 MPa, and the reaction was stirred overnight at room temperature. After the complete reaction monitored by TLC, the resulting mixture was filtered, washed with THF, and concentrated to obtain a crude product. The crude product was purified by column chromatography to obtain A7, with a yield of 85%.

### Step 5:

Compound A7 was dissolved in DCM and cooled to 0-5°C, and Dess-Martin oxidant was added in portions thereto. The reaction was strirred for 2-4 hours at 0-5 °C room temperature. After the reaction is completed, the reaction was quenched with a mixed solution of sodium bicarbonate and sodium sulfite. The organic phase was washed with saturated sodium bicarbonate aqueous solution, then washed again with saturated saline solution, and subjected to vacuum rotary evaporation to obtain a crude product. The crude product was purified by column chromatography to obtain A8, with a yield of 70%.

### Example 2

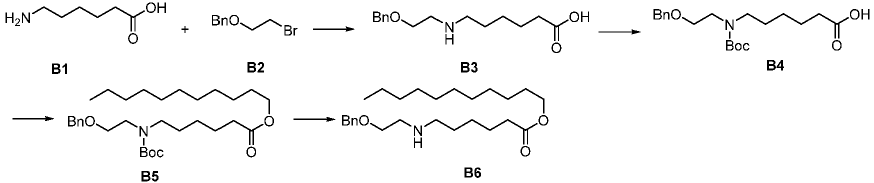

### Step 1:

6-Aminocaproic acid (B1, 3.5 kg) was dissolved in 21.8 kg of ethanol, and 3.0 kg of potassium hydroxide was added in portions thereto. Benzyloxybromoethane (6.8 kg) was then added, and the reaction was stirred for 20 hours at 50-60 °C. The resulting mixture was cooled, filtered, and concentrated under reduced pressure to remove most of the ethanol. Methyl tert-butyl ether and water were added, and the pH was adjusted to 6. After the solution was partitioned, the aqueous layer was retained, and washed further with methyl tert-butyl ether and dichloromethane respectively. The aqueous phase was directly used for the next step.

### Step 2:

The solution obtained in the previous step was added to 1,4-dioxane (22.5 kg), and 50% NaOH (1.46 kg) aqueous solution was added. Boc₂O (4.23 kg) was slowly added dropwise, and the reaction was stirred. The resulting mixture was concentrated to remove dioxane, and water was added and washed twice with methyl tert-butyl ether. The pH was adjusted to 5-6 with 2N hydrochloric acid. The resulting mixture was extracted twice with methyl tert-butyl ether, and the organic phases were washed with 5% NaHCO₃ aq. The organic phases were combined, filtered, and the filtrate was concentrated to obtain B4 as an oil, with an estimated two-step yield of 30%.

### Step 3:

Compound B4 was dissolved in dichloromethane (26 kg), and undecanol (0.94 kg), EDCI (1.57 kg), DIPEA (1.77 kg) and DMAP (0.17 kg) were added in portions thereto. The reaction was stirrred for 14-18 hours at room temperature. Purified water (15 kg) was added, and the solution was partitioned, washed with 1% citric acid and 3% citric acid. The organic phase was washed with saturated NaCl (10.0 kg), and the solution was partitioned, filtered, and concentrated to dryness to obtain B5 as an oil, with a yield of 73%.

### Step 4:

Compound A5 (2.8 kg) was dissolved in acetonitrile (22.0 kg, 10 V) and stirred well. A solution (5.3 L) of 4M HCl in dioxane was added thereto. The reaction was performed for 1-4 hours at room temperature. The resulting mixture was cooled down to -10-0°C, and stirred to precipitate a solid. The solid was filtered, washed with methyl tert-butyl ether, and subjected to vacuum drying at 40°C to obtain B6 as a white solid, with a yield of 72%.

### Example 3

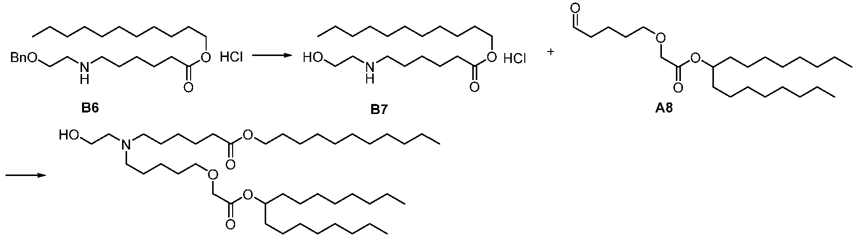

### Step 1:

Compound B6 (2.0 kg) was dissolved in ethanol (16 kg, 10V) and stirred until clear. Wet 10% palladium on carbon (0.4 kg, 20% wt.) was added and the reaction system was subjected to vacuum/hydrogen replacement three times. The reaction system was heated to 40 ± 5 °C and stirring was continued for 8-10 hours. The resulting mixture was filtered and washed with ethanol. The filtrate was concentrated under reduced pressure, cooled to precipitate a solid, while stirring was continued for 4-6 hours. After filtration, the filter cake was washed with methyl tert-butyl ether. The filter cake was subjected to vacuum drying for 16 hours to obtain B7 as a white solid, with a yield of 90%.

### Step 2:

Compound A8 (665 g) was dissolved in dichloromethane (10 kg, 12V). B7 (550 g) was added in portions, and then *N, N*-diisopropylethylamine (324 g) and acetic acid (202 g) were added successively. The resulting mixture was cooled to 0-5°C. Sodium triacetoxyborohydride (887 g) was added in portions. The reaction was stirred for 2-4 hours, and then was quenched by adding purified water. The organic phase was washed with sodium bicarbonate aqueous solution and sodium chloride aqueous solution respectively, and concentrated under reduced pressure.

Methanol was added and stirred until clear. A powder of activated carbon (40 g, 5% wt) was added to decolorize. The resulting mixture was kept at 30 °C and stirred for 1 hour. After filtration and concentration, the crude product was purified by column chromatography to obtain a target product with a yield of 80%.

## Claims

1. A method for preparing a compound of formula I or a pharmaceutically acceptable salt thereof, 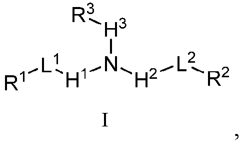 comprising a step of reacting a compound of formula A with a compound of formula B to form a compound of formula I, 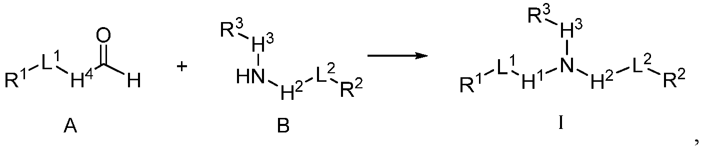 wherein L¹ and L² are each independently selected from the group consisting of -C(O)O-, -OC(O)-, -C(O)- and -OC(O)O-;
H¹ and H² are each independently selected from the group consisting of C₁₋₁₂ heteroalkylene, C₁₋₁₂ alkylene and C₂₋₁₂ alkenylene;
H³ is selected from the group consisting of C₁₋₂₄ alkylene, C₂₋₂₄ alkenylene, C₃₋₈ cycloalkylene and C₃₋₈ cycloalkenylene;
H⁴ is selected from the group consisting of C₂₋₁₁ heteroalkylene, C₂₋₁₁ alkyleneand C₃₋₁₁ alkenylene;
R¹ and R² are each independently selected from the group consisting of C₁₋₂₄ alkyl and C₂₋₂₄ alkenyl;
R³ is selected from the group consisting of -CN, -C(O)OR⁴, -OC(O)R⁴, -OR⁵ and -NR⁵C(O)R⁴, R⁴ is selected from the group consisting of C₁₋₆ alkyl and C₂₋₆ alkenyl, and R⁵ is selected from the group consisting of hydrogen, C₁₋₆ alkyl and C₂₋₆ alkenyl.

2. The method according to claim 1, wherein the compound of formula A is reacted with the compound of formula B in the presence of a reducing agent selected from the group consisting of sodium borohydride and sodium triacetoxyborohydride.

3. The method according to claims 1 or 2, wherein a base is also included, and the base is preferably *N, N*-diisopropylethylamine.

4. The method according to any one of claims 1-3, wherein the compound of formula I is a compound of formula I-1, 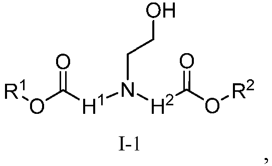 and the method comprises a step of reacting a compound of formula A-1 with a compound of formula B-1, 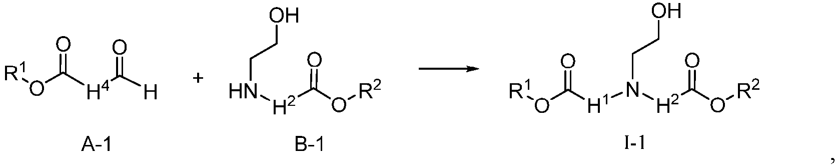 wherein H¹, H², H⁴, R¹ and R² are as defined in claim 1.

5. The method according to any one of claims 1-4, wherein at least one of H¹ and H² in the compound of formula I is C₁₋₁₂ heteroalkylene, preferably C₂₋₉ heteroalkylene, more preferably C₂₋₅ heteroalkylene.

6. The method according to any one of claims 1-5, wherein R¹ is selected from the group consisting of 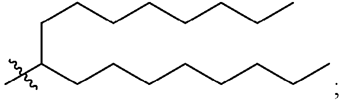 H¹ is selected from the group consisting of C₂₋₉ heteroalkylene.

7. The method according to any one of claims 1-5, wherein R² is selected from the group consisting of 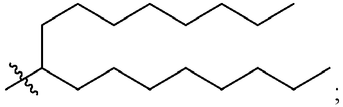 H² is selected from the group consisting of C₂₋₉ heteroalkylene.

8. The method according to any one of claims 1-5, wherein the compound of formula I is a compound of formula I-2, 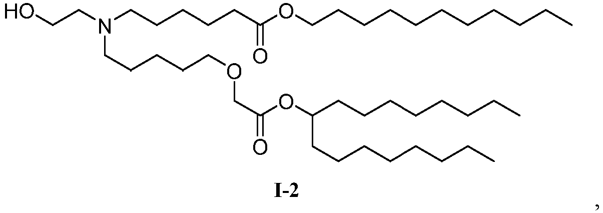 and the method comprises a step of reacting a compound of formula A-2a with a compound of formula B-2a, 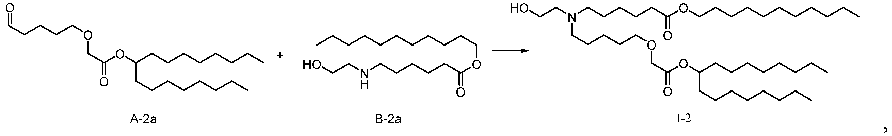 or, a step of reacting a compound of formula A-2b with a compound of formula B-2b,

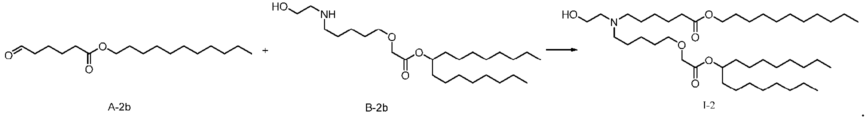

9. The method according to claim 8, wherein the method also comprises a step of converting a compound of formula B-2a5 to the compound of formula B-2a, 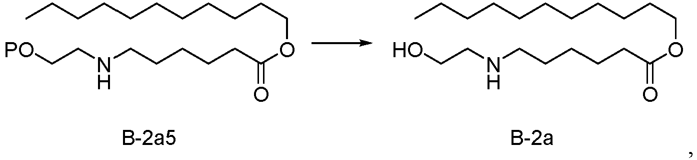 wherein, P is a protecting group for hydroxy, for example a benzyl or benzoyl group.

10. The method according to claim 9, wherein the condition for removing the protecting group for hydroxy is a hydrogenation condition, preferably comprising a Pd/C catalyst.

11. The method according to any one of claims 1-10, comprising forming the compound of formula B-2a from the compound of formula B-2a5 under a hydrogenation condition, and subsequently reacting the compound of formula B-2a with a compound of formula A-2a to form a compound of formula I-2,

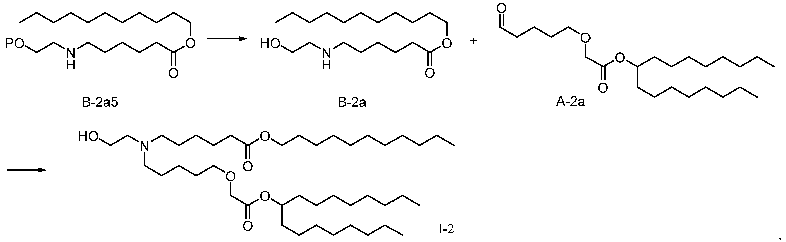

12. A pharmaceutical composition comprising a compound of formula I or a pharmaceutically acceptable salt thereof prepared by the method of any one of claims 1-11, and one or more pharmaceutically acceptable excipients.

13. The pharmaceutical composition according to claim 12, further comprising an active agent, preferably a polynucleotide or nucleic acid such as mRNA.

14. Use of a compound of formula I or a pharmaceutically acceptable salt thereof prepared by the method of any one of claims 1-11 or the pharmaceutical composition according to claim 12 in the preparation of a medicament for the prevention and/or treatment of an immune response induced in a subject.

15. Use of a compound of formula I or a pharmaceutically acceptable salt thereof prepared by the method of any one of claims 1-11 or the pharmaceutical composition according to claim 12 in the preparation of a medicament for the prevention and/or treatment of a disease or disorder related to overexpression of polypeptide.

16. Use of a compound of formula I or a pharmaceutically acceptable salt thereof prepared by the method of any one of claims 1-11 or the pharmaceutical composition according to claim 12 in the preparation of a medicament for the prevention and/or treatment of a disease or disorder related to underexpression of polypeptide.
